# EUROPEAN PATENT SPECIFICATION

(11) **EP 4 161 414 B1**
(45) Date of publication and mention of the grant of the patent: **10.12.2025**
(21) Application number: 21729797.7
(22) Date of filing: 03.06.2021
(51) Int. Cl.: A61B 17/43, A61B 10/00

(54) **SYSTEM FOR ASSISTING CONCEPTION**
SYSTEM ZUR EMPFÄNGNISUNTERSTÜTZUNG
SYSTÈME D'ASSISTANCE À LA CONCEPTION

(30) Priority: 03.06.2020 GB 202008361
(43) Date of publication of application: 12.04.2023
(73) Proprietor: Stepone Fertility Limited, St. John's Hill London SW11 1SN (GB)
(72) Inventor: COSAD, Tess Isabelle, London SW11 1SN (GB); O'ROURKE, David Gerard, London SW11 1SN (GB)
(74) Representative: Håmsø Patentbyrå AS
(86) International application number: PCT/EP2021/064945
(87) International publication number: WO 2021/245205

(56) References cited:
- CN-A- 106 308 899
- US-A1- 2012 310 040
- US-A1- 2015 196 324
- US-A1- 2018 014 854
- THE STORK OTC: "How to use The Stork OTC", VIMEO, 12 September 2016 (2016-09-12), pages 1, XP054982152, Retrieved from the Internet <URL:https://vimeo.com/182438539> [retrieved on 20210819]

## Description

### FIELD

The present invention relates to a system for assisting conception and a method of arranging the system for assisting conception.

### BACKGROUND

Assisted conception has been used in humans and animals for many decades. Assisted conception is the general group of procedures and processes of delivering semen comprising many millions of sperm to a cervix or uterine cavity with the aim of achieving a pregnancy. There are different types of assisted conception including intrauterine insemination and intracervical insemination.

Many attempts have been made to devise a device to assist in delivering semen to the cervix. Most notably, the device described in US patent application US 2012/0310040A1 includes a receptacle for receiving sperm from a penis, a delivery device for loading the receptacle into and temporarily closing the receptacle whilst in transit to the cervix, and a reservoir inverter to propel the semen from the base of the receptacle into a cap and on to the cervix. This device has a complicated internal structure to achieve sealing of the receptacle and protection of the receptacle as it is in transit to the cervix. This can lead to failure of the device and a missed cycle which can be very frustrating for the user. Furthermore, the device can often lead to a failed attempt at conception as semen can easily be lost after being gathered in the receptable, as the user must attach the receptacle to the delivery device so that the receptable can be positioned, by the delivery device, inside the vagina and at the cervix. A product called Stork OTC available from Rinovum Women's Health of Pittsburgh, Pennsylvania, also encounters the above-mentioned problems of complex internal mechanism and often accidental loss of semen.

Attempts have also been made to create simpler devices to assist in the positioning of quantities of semen at the cervix; one such device being that described in US patent application US 2010/0242968A1. This, and other manually positioned devices, are difficult for the user to position correctly and awkward for the user to use in a domestic environment. Furthermore, these later devices may also suffer from accidental spillage of semen as the receptacle is often open at the top during transfer of semen thereto, and/or at least partially open during insertion of the device into the vagina.

United States patent document US 2015/196324A1 discloses an object sized and shaped for insertion and deposition in the vaginal tract of a human comprising a removal string wherein: the removal string has a first end and a second end; the first and second ends are both attached to the object; and the string is of sufficient length that when the object is placed in the vaginal tract at least a portion of the string may protrude from the vagina.

The present invention serves to overcome at least some of the aforementioned problems.

### SUMMARY

The invention is defined by the independent claims. A selection of optional features is provided by the dependent claims.

### BRIEF DESCRIPTION OF THE DRAWINGS

Embodiments of the invention will now be described with reference to the following drawings, in which:
Fig. 1a shows a cross-sectional view of a system according to the first aspect of the invention in the loading configuration, with the implant not shown for clarity;
Fig. 1b shows the system of Fig. 1;
Fig 1c, 1d and 1e show a rotatable hoop;
Fig 1f and 1g show the system at different tilt angles;
Fig. 2a shows a cross-sectional view of the threaded member of the release mechanism within the housing of the system shown in Fig. 1a;
Fig 2b shows the implant actuator protruding from the housing;
Figs. 3a and 3b show the implant of the system of Fig. 1a;
Fig. 4a shows the implant and the funnel of the system of Fig. 1a;
Fig. 4b shows the cap of the system of Fig. 1a being folded;
Fig. 4c shows the cap of the system of Fig. 1a inserted within the annulus between the sleeve and the channel portion of the funnel;
Figs. 5a and 5b show the system of Fig. 1a in the delivery configuration;
Fig. 5c shows the system of Fig. 1a moving from the delivery configuration to the open configuration;
Figs. 6a, 6b and 6c show the system of Fig. 1a moving from the delivery configuration to the open configuration;
Fig. 7 shows the system of Fig. 1a in the open configuration with the implant released and separated from the housing;
Figs 8a and 8b show an alternative implant threaded in a first manner;
Figs 8c and 8d show the alternative implant of Figs 8a and 8b threaded in a second manner;
Figs 9a-9e show an alternative implant comprising apertures located in the cap;
Figs 10a-10d show an alternative implant;
Fig 10e shows an implant actuator for use with the implant of Figs 10a-10d; and
Fig 10f shows a system for use with the implant of Figs 10a-10d.

### DETAILED DESCRIPTION OF THE DRAWINGS

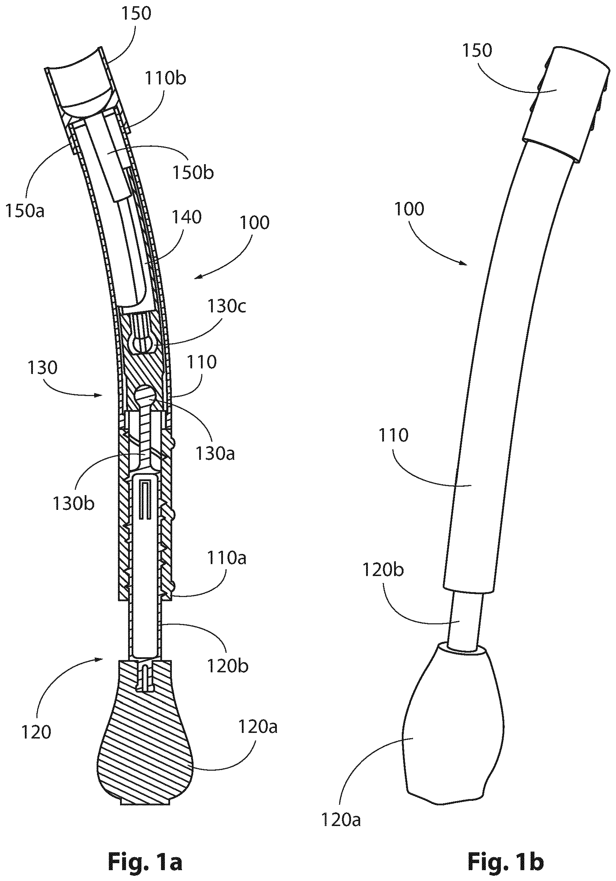

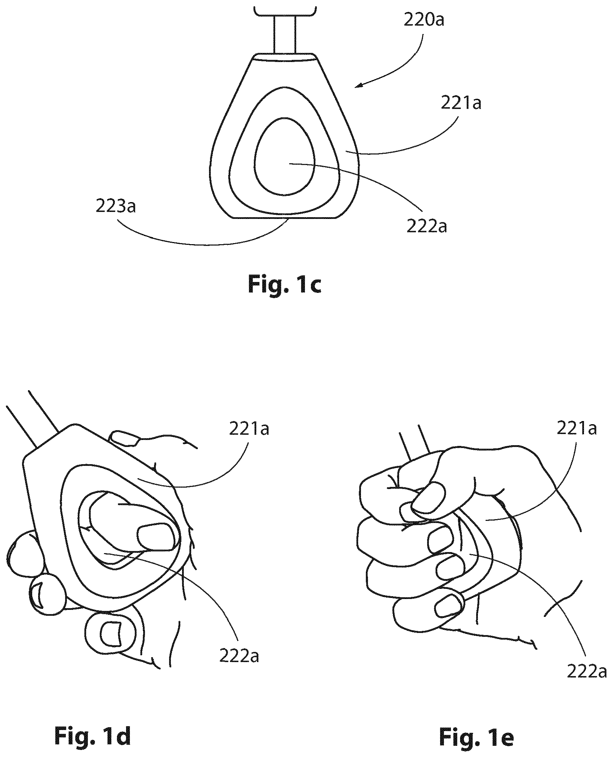

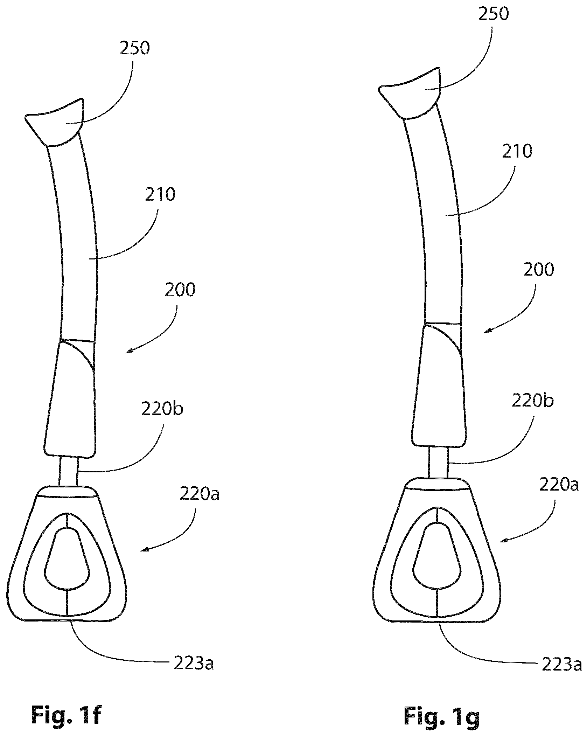

Fig. 1 shows a system 100 in accordance with an embodiment of the present invention. The system 100 can be broadly described to be moveable between three configurations: the loading configuration; the delivery configuration and the open configuration. The loading configuration is used to load a semen sample into an implant within the system 100. The delivery configuration is used when the system 100 is inserted into a vagina. The open configuration is used when the system 100 is positioned at the cervix, and the semen is to be released from the implant on to or adjacent the cervix. The system 100 is shown in Fig. 1a in a loading configuration.

The system 100 comprises an elongate curved housing 110 in the form of an open-ended tubular. The housing 110 has a proximal end 110a and a distal end 110b. The housing 110 provides internal space within which the implant (not shown) and other mechanisms can be safely housed and protected during insertion of the system 100 inside a vagina. The housing 110 has a slight curve to allow the distal end 110b to reach the cervix when the housing 110 is in the vagina.

The implant (not shown) is securely enclosed within the housing 110 near to the distal end 110b of the housing 110, as will be explained further with reference to the later drawings.

The housing 110 has a release mechanism 120 attached at its proximal end 110a, which in the present embodiment is a rotatable knob 120a and a threaded member 120b. The rotatable knob 120a can optionally have a flat bottom or feet arranged such that the system 100 can stand upright freely. It will be appreciated that the release mechanism 120 may be any other suitable device which can be easily operated be a user, such as a plunger or push button. The threaded member 120b comprises thread projections or recesses which register with corresponding recesses or thread projections on the internal surface of the housing 110 toward its proximal end 110a. This arrangement allows the knob 120a to be rotated by a user to cause the threaded member 120b to travel into the housing 110 when the user wants to move the system 100 to an open configuration, i.e. when the distal end 110b of the housing 110 is located adjacent the cervix. The movement of the threaded member 120b as above described causes the implant to be moved out of the housing 110 by utilising a connection with an intermediate arrangement which is arranged to allow movement through the curved section of the housing 110.

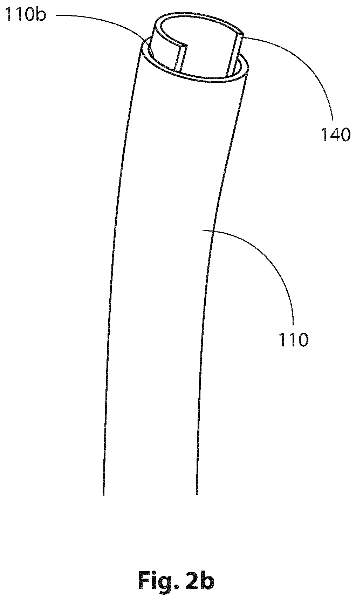

In this regard, the system 100 comprises a ball-and-socket joint 130 to serve as the intermediate arrangement, wherein a ball 130a is connected by a shaft 130b to the threaded member 120b, to allow the travel of the threaded member 120b into the housing 110 to push the shaft 130b and the ball 130a. A corresponding socket 130c mates with the ball 130a to allow multi-directional movement. The socket 130c is attached to an implant actuator 140 which is shaped and arranged to slide within the housing 110 such that the implant actuator 140 will push the implant out of the housing 110 as the user turns the rotatable knob 120a. As shown in more detail in Fig 2b, the implant actuator 140 is arranged to protrude from the distal end 110b of the housing 110 when the rotatable knob 120a is turned, thereby ensuring that the implant is pushed out of the housing 110.

Still referring to the system 100 in the loading configuration in Fig. 1a, the system 100 further comprises a removeable funnel 150 attached to the distal end of the housing 110. The funnel 150 is provided to allow efficient transfer of semen from outside of the system 100 to inside the system 100, and specifically, as will be described, directly to the implant.

The funnel 150 comprises a sleeve portion 150a and a channel portion 150b. The sleeve portion 150a is sized and arranged to register with the distal end 110b of the housing 110 such that the funnel 150 can be attached to the housing 110 via the sleeve portion 150a. The channel portion 150b is concentrically arranged with the sleeve portion 150a such that, as shown in Fig. 1a, when the sleeve portion 150a is attached to the distal end 110b of the housing 110, the channel portion 150b protrudes into the housing 110 and is concentric with the distal end 110b of the housing 110. The channel portion 150b may be substantially circular in cross-section or may be oval shaped. The sleeve portion 150a and the distal end 110b of the housing 110 may be configured to engage releasably in any known manner, such as, but not limited to, correspondingly registered threads or an interference fit.

In the loading configuration shown in Fig. 1a, the implant (not shown) is attached to the end of the channel portion 150b, such that the user can transfer semen to the implant by pouring semen from a receptacle or ejaculating semen into the funnel 150.

The direct transfer of semen to the implant held within the housing 110 mitigates the risk of losing all or part of a semen sample when compared with arrangements which require the implant to be filled outside of the housing 110 and then the implant transferred to the housing 110 for delivery to the cervix. The present invention overcomes accidental spillage of semen and/or total loss of a semen sample as the implant is transferred to the housing 110. The present invention also greatly simplifies the loading operation by only requiring the user to provide a semen sample to the funnel 150 and then remove the funnel 150 from the housing 110. Other advantages will also become apparent with reference to the later drawings.

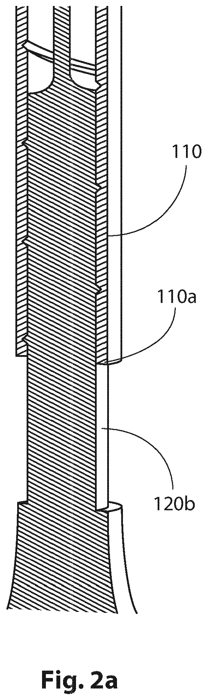

Components of the system 100 can also be seen in Figs. 1b and 2. Fig. 1b shows the rotatable knob 120a, the threaded member 120b within the housing 110 and the removeable funnel 150, whilst Fig. 2 shows a detailed view of the threaded member 120b comprising threads which are registered with corresponding threads on the internal surface of the housing 110 near the proximal end 110a.

In an alternative example shown in Figs 1c-1e, the rotatable knob 120a is replaced by a rotatable hoop 220a. Firstly referring to Fig 1c, the rotatable hoop 220a comprises a solid or hollow ring portion 221a forming a central aperture 222a. The ring portion 221a comprises a flat bottom 223a which is configured to allow the system 200 to easily stand on a flat surface such as a bedside table, desk or work surface. As shown in Figs 1d and 1e, the rotatable hoop 220a provides a particularly convenient ergonomic grip which allows the user to place a finger through the central aperture 222a or grip the outside of the ring portion 221a. The hoop 220a is a particularly convenient shape to allow rapid injection moulding.

The alternative rotatable hoop 220a is shown arranged as part of a system 200 similar to the system 100, in Figs. 1f and 1g, with like reference numerals with the addition of 100 indicating like parts. In Figs 1f and 1g, the housing 210 is arranged to have a 4 degree tilt and 2 degree tilt respectively, with respect to the threaded member 220b. A small degree tilt between the threaded member 220b and the housing 210 moves the centre of gravity of the system 200 such that it is less likely to easily tip over when standing upright on the rotatable hoop 220a. Additionally, the small degree of tilt allows the funnel 250 to sit substantially upright from the top of the housing 210. This allows easier collection and/or loading of the semen sample into the system 200, thereby reducing the risk of a spillage or loss of the semen sample. Furthermore, a substantially upright housing 210 allows the funnel 250 to be removed easily without undesirable tipping of the funnel 250 as it is removed from the housing 210. This ensures any remaining semen in the funnel 250 does not spill. Additionally, the small degree of tilt allows the funnel 250 to be removed in a substantially upright fashion, thereby reducing the risk that the funnel 250 will catch the reservoir 160b of the implant 160 at an angle and thereby drag the implant 160 out of the housing 110.

In the described examples the angle of tilt is 4 degrees in Fig 1f and 2 degrees in Fig 1g, however it will be understood that any small degree of tilt may be used depending on the particular design and configuration employed by the system 200. For example, a less than 1 degree of tilt may be sufficient. Alternatively, any degree of tilt between 1 degree and 30 degrees may be provided. Typically, between 2 degrees and 4 degrees achieves the discussed effect.

It will be understood that in other examples the degree of tilt may be provided between the rotatable hoop 220a and the threaded member 220b. Alternatively, the flat bottom 223a may be provided at an angle to provide tilt to the system 200 when the system 200 is standing upright, thereby providing the desired stabilising of the system 200 and uprighting of the funnel 250.

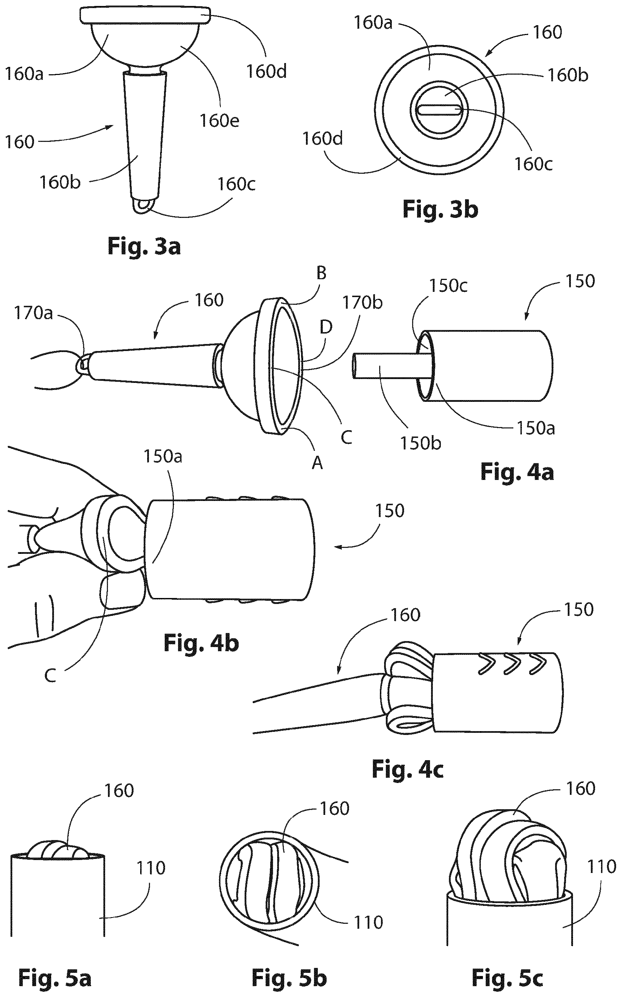

Referring now to Figs. 3a and 3b, which show the implant 160 of the presently described embodiment of the system 100 in side and plan views, respectively. The implant 160 comprises a cap 160a and a reservoir 160b. The cap 160a is sized and arranged to be positioned at the cervix, and is fluidly connected to the reservoir 160b which is arranged to hold semen and is sufficiently resilient such that semen can be expelled therefrom and into the cap 160a by pressure from the vaginal walls of the user pressing on the outer surfaces of the reservoir 160b when the implant 160 is installed adjacent the cervix.

The implant 160 further comprises a retainer loop 160c for receiving a retrieval thread (not shown) therethrough. It will be appreciated that the implant 160 may be retrieved in other ways and using other means as appropriate for the user.

The cap 160a comprises a circular rim 160d and a hemispherical shell 160e. The cap 160a is made is a resilient material, such as, but not limited to, medical grade silicone. The rim 160d is of greater stiffness than the hemispherical shell 160e, such that the rim 160d has sufficient rigidity compared to the shell 160e to allow the rim 160d to be folded by a finger into a position in which the rim 160d is biased whilst the shell 160e simply folds. The shell 160e is sufficiently resilient such that whenever it is folded or compressed by the folding of the rim 160d, the shell 160e is able to return to the position shown in Figs. 3a and 3b, i.e. an open and unfolded hemispherical shell.

Referring now to Fig. 4a, 4b and 4c, a method of arranging the implant 160 and funnel 150 for providing the system 100 in the loading configuration is now described. The funnel 150 and implant 160 are arranged together outside of the housing 110, typically by the manufacturer of the system 100 before the system 100 is sent to the user for loading with semen and delivery to the cervix. Preferably, the system 100 is single use. In this regard, the user is instructed to discard of the system 100 after use. To improve the chance of conception, the user may be provided with two systems 100 from the manufacturer, thus allowing a repetition of the process on both fertile days of a monthly cycle. The system 100 may comprise a means of ensuring single use either by the arrangement of the components or by a separate feature which activates to eliminate reuse of the system 100. The feature (not shown) may be any commonly used feature in medical devices such as, but not exclusively, a breakable element or locking pin. However, instead of the system 100 being arranged solely by the manufacturer, the system 100 may also be arranged by the user of the system 100 if for example the user wishes to perform a second cycle of deploying the same implant 160 at the cervix and a system 100 suitable for multiple uses has been provided. In this regard, the user may perform a first cycle, retrieving the implant 160 using the retrieval thread as previously described, and then configure the system 100 in the loading configuration again such that it is ready to receive a second semen sample. The method of arranging the system 100 in the loading configuration is now described.

As shown in Fig. 4a, the implant 160 comprises a proximal end 170a and a distal end 170b. In the loading configuration shown in Figs. 1a and 1b, the implant 160 is aligned longitudinally within the housing 110. Still referring to Fig. 4a, the implant 160 is brought into attachment with the funnel 150, such that the channel portion 150b passes through the inside of the rim 160d and the shell 160e to attach to the reservoir 160b by sliding into the top of the reservoir 160b. The resilient material of the implant 160 allows the reservoir 160b to expand radially to grip the channel portion 150b as it is inserted into the end of the reservoir 160b.

Still referring to Fig. 4a, the cap 160a of the implant 160 is then folded and partially inserted within an annulus 150c between the sleeve 150a and the channel portion 150b. The cap 160a is firstly folded by pressing two diametrically opposite points A, B on the rim 160d toward each other. The cap 160a is then folded further by pushing two other diametrically opposite points C, D on the rim towards the proximal end 170a of the implant 160.

Fig. 4b shows the cap 160a being folded by two fingers pressing points A and B towards each other, and tucking points A and B into the inner surface of the sleeve 150a to allow the sleeve 150a to grip points A and B while the assembler then pushes points C and D towards the proximal end 170a of the implant 160, and then, as shown in Fig. 4c, the assembler slides the cap 160a into the annulus 150c. The step of tucking points A and B into the inner surface of the sleeve 150a to allow points C and D to be pushed back towards the proximal end 170a may not be required if the assembler can hold A and B together whilst pushing C and D. The combined effects of pushing A and B together, either tucking A and B into the sleeve 150a or holding A and B in the pushed together position, pushing points C and D towards the proximal end 170a, and crucially sliding the cap 160a into the annulus 150c, creates the desired biasing at the distal end 170b of the implant 160. That is to say the distal end 170b of the implant 160 is biased towards the channel portion 150b of the funnel 150. Preferably, however not essentially, the channel portion 150b may be oval in cross section to assist the folding steps by providing a larger surface on two opposite sides of the channel portion 150b.

As the folded cap 160a is pushed into the annulus 150c, the channel portion 150b is pushed further into the reservoir 160b, further securing the channel portion 150b there within.

The folded cap 160a can be considered to be a clam shell arrangement, in that when points C and D are pushed back towards the proximal end 170a, the distal end 170b of the implant 160 comprises two curved portions which come together around the channel portion 150c, such that when the channel portion is removed, as will later be described, the two curved portions come together and seal the implant 160, in a similar fashion to the closing of a clam shell.

Once the cap 160a has been folded and inserted within the channel 150c, the funnel 150 is attached to the distal end 110b of the housing 110 by the sleeve 150a as previously described. This positions the implant 160 within the housing 110 where it can be loaded with semen via the funnel 150, and deployed from within the housing 110 by the implant actuator 140.

As the funnel 150 is attached to the housing 110, the distal end 110b of the housing 110 comes between the sleeve 150a and the implant 160, thus transferring the implant 160 to the housing 110 and biasing the proximal end 170a of the implant 160 against the housing 110 so that it is now positioned within and enclosed by the housing 110, albeit with the channel portion 150b still attached to allow the loading of semen into the reservoir 160b. In this connection, semen may be collected by male masturbation into an appropriate receptacle or may be available for use at a sperm bank. When sperm is provided by a sperm bank, the sperm can be delivered to the funnel by a straw or vial from the sperm bank. Any other technique of collecting or storing sperm known in the field of assisted conception may be used prior to transfer to the funnel 150. Typically, an appropriate receptacle may be required to catch and contain the semen sample after ejaculation before transfer either to a sperm bank for storage or before direct transfer to the funnel 150. In this regard, a sterile collection pot (not shown) can be used. Alternatively, ejaculation may be performed directly into the funnel 150 to allow direct transfer of semen into the funnel 150. Regardless of the method in which semen enters the funnel 150, the semen will transfer to the reservoir 160b. When all of the semen has transferred, the user can release the funnel 150 from attachment with the housing 110, thus pulling the channel portion 150b out of the reservoir 160b. As the funnel 150 is removed, the channel portion 150b will also be disengaged from the rim 160d of the cap 150a. As previously described, when the channel portion 150b is so removed, the rim 160d will form a closed clam shell arrangement, with points A and B touching to seal the implant 160 closed, such that no semen can escape from it. The system 100 is now in the delivery configuration, and the system 100 (without the funnel 150) can be inserted into a vagina such that the distal end 110b arrives adjacent the cervix.

The folded clam shell arrangement biases points C and D outward against the housing 110, whilst points A and B are biased towards each other to maintain sealing of the clam shell.

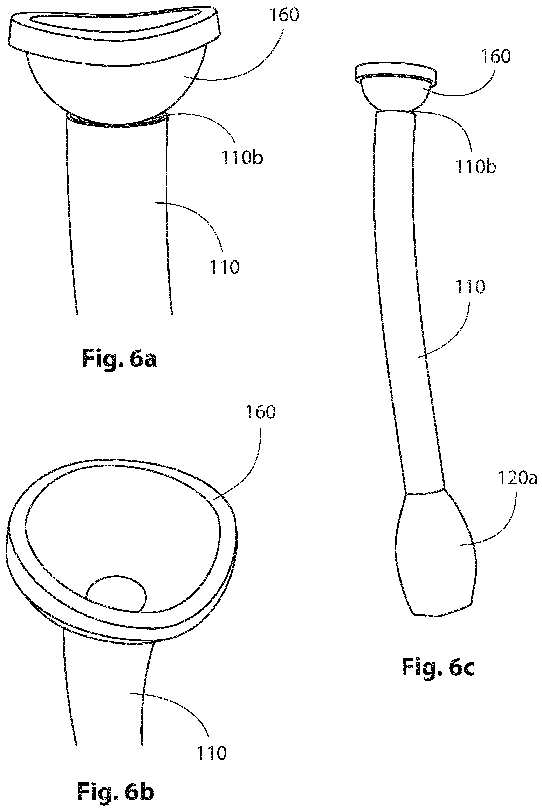

This biasing arrangement is advantageous when the system 100 is to be moved from the delivery configuration to the open configuration, as will now be described with reference to Figs. 5a-5c and 6a-6c. Referring firstly to Figs. 5a and 5b, the system 100 is shown in the delivery configuration with the implant 160 sealed closed. In the presently described embodiment not forming part of the invention, the implant 160 protrudes from the housing 110 in this configuration, however, according to the invention, it is fully enclosed within the housing 110 and not protruding therefrom. As shown in Fig. 5c, the implant 160 protrudes further from and comes out from enclosure within the housing 110 as the user turns the rotatable knob to move the system 100 from the delivery configuration to the open configuration. The biasing of points C and D towards the housing 110 ensures that as points C and D pass the distal end 110b of the housing 110, the implant 160 will automatically open as the resiliency of the material causes the cap 160a to return to a hemispherical shell shape with a circular rim, as shown in Figs. 6a-6c.

Still referring to Figs. 6a-6c, the implant 160 is now in position adjacent the cervix. The vaginal walls are closed around the housing 110 which allows the housing 110 to be removed by pulling the housing 110 from the vagina whilst the implant 160 is stopped from also being pulled out as the implant 160 is no longer attached to the housing 110 and the opened hemispherical shell 160e provides resistance against being pulled through the vaginal walls, ensuring the implant 160 is held at the cervix.

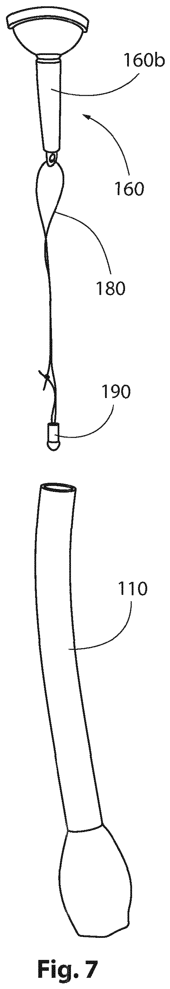

The system 100 is shown in the open configuration in Fig. 7, where it can be seen that the housing 110 has completely detached from the implant 160. Although not show in Fig. 7, the implant 160 is in position within the vagina and the vaginal walls are pressing against the reservoir 160b to expel semen from the reservoir 160b into the cap 160a and onto the cervix, increasing the chance of sperm within the semen entering the uterus and causing conception. The retrieval thread 180 has also been deployed from the housing 110 and provides a means for the user to pull the implant 160 from outside of the vagina when the user wants to remove the implant 160. In this regard, the retrieval thread 180 has a bulb 190 which can be gripped by the user when pulling the retrieval thread 180.

It will be understood that while the funnel 150 is used in the described embodiment, other fluid channelling and delivery devices may be suitably adapted to serve the same purpose. In this regard, is it envisaged that the funnel 150 may be replaced by a receptacle comprising the described features, or a medical syringe, or any other device adapted to perform the functions of attachment to the distal end 110b of the housing 110 and fluid communication via a channel portion 150b with the reservoir 160b.

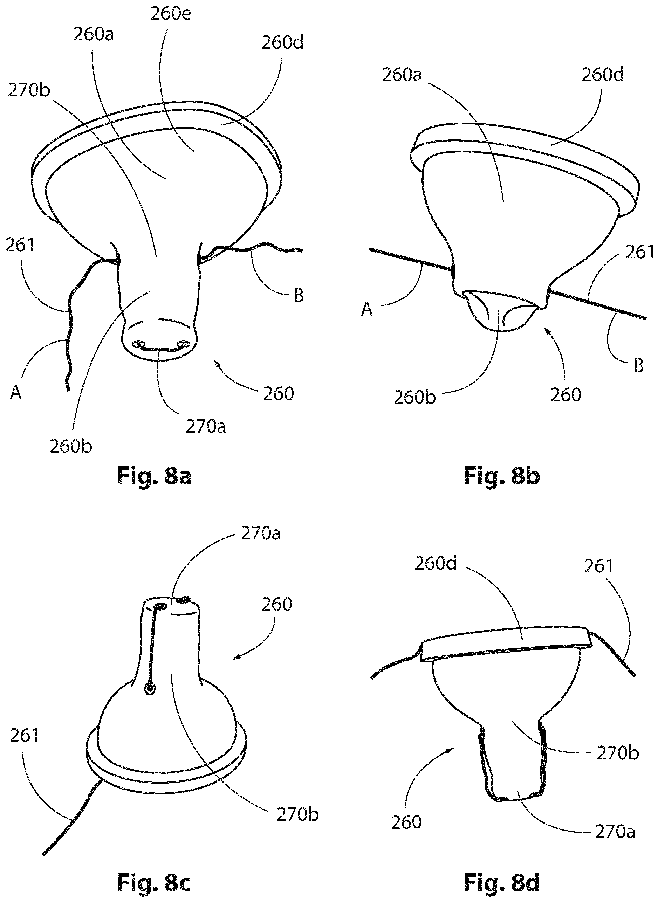

Referring now to Figs. 8a to 8d, which show an alternative example of an implant 260. The implant 260 is similar to the implant 160 shown in Figs 3a and 3b, with like reference numerals with the addition of 100 indicating like parts. The implant 260 comprises a cap 260a and a reservoir 260b. The cap 260a is sized and arranged to be positioned at the cervix, and is fluidly connected to the reservoir 260b which is arranged to hold semen and is sufficiently resilient such that semen can be expelled therefrom and into the cap 260a by deformation of the reservoir 260b when the implant 260 is installed adjacent the cervix, as will now be explained.

The implant 260 comprises a string 261 which is arranged to compress the reservoir 260b when the string is simultaneously pulled on both a first side A and a second side B. In the presently described example the string 261 is configured to compress the reservoir 260b by applying a force to the proximal end 270a of the reservoir 260b, the force tending to compress the reservoir 260b by pulling the proximal end 270a of the reservoir 260b towards the distal end 270b, as shown in Fig 8b. In this way, semen within the reservoir 260b is ejected out of the reservoir 260b. The string 261 may be arranged to be threaded through the implant in various ways including a first example shown in Figs 8a and 8b and a second example shown in Figs 8c and 8d. The cap 260a comprises a circular rim 260d and a hemispherical shell 260e. The cap 260a is made is a resilient material, such as, but not limited to, medical grade silicone. The rim 260d is of greater stiffness than the hemispherical shell 260e, such that the rim 260d has sufficient rigidity compared to the shell 260e to allow the rim 260d to be folded by a finger into a position in which the rim 260d is biased whilst the shell 260e simply folds. The shell 260e is sufficiently resilient such that whenever it is folded or compressed by the folding of the rim 260d, the shell 260e is able to return to the position shown in Figs. 8a-8d, i.e. an open and unfolded hemispherical shell.

In the first example shown in Figs 8a and 8b, the string 261 may be pulled to compress the reservoir 260b by the user pulling both sides A, B of the string 261 simultaneously allowing the user to deploy the implant 260 and then compress the reservoir 260b in two separate steps. Alternatively, the string 261 may be attached to the rotatable knob 120a such that turning of the rotatable knob 120a pulls sides A and B of the string 261, thereby allowing deployment of the implant 260 and compression of the reservoir 260b simultaneously. It will be understood that the string 261 may be attached to another manual or automatic system arranged to pull both sides A, B of the string 261 when required. In alternative examples not shown, the string 261 may be wrapped around the reservoir 260b in such a way as to allow compression of the reservoir 260b by pulling on one side of the string 261.

In an alternative example shown in Figs 8c and 8d, the string 261 is routed through the aperture formed by the rim 260d. The string 261 is arranged to compress the reservoir 260b when the rim 260d applies force to both sides of the string 261 simultaneously. The string 261 passes through the rim 260d and through the distal end 270b of the reservoir 260b and travels along the outside of the reservoir 260b before returning inside the reservoir 260b at the proximal end 270a. In this arrangement the string 261 is taught (not shown) over the rim 260d and may be held by the user or held by another component located on the housing (not shown) or elsewhere in the system. Although not shown in the drawings, using this arrangement provides automatic pulling of the string 261 as the implant 260 is deployed, as will now be described. As the cap 260a opens from the previously described clamshell configuration (shown in Figs 5a-5c) the taught string 261 is pushed by the rim 260d. The rim 260d may be significantly stiffer than the reservoir 260b, thereby ensuring that the rim 260d pushes the string 261 as the cap 260a expands, and in doing so compresses the reservoir 260b to eject semen from the reservoir 260b. The implant 260 may also be arranged such that the string 261 threaded in the manner shown in Figs 8c and 8d may be pulled by the user to compress the reservoir 260b rather than by the described automatic operation. To this end, in use the string 261 would be gently pulled by the user, with the shape of the cap 260a restricting the implant 260 from being easily pulled out through the vaginal walls, thereby ensuring that the string 261 does not pull the implant 260 away from the cervix and instead compresses the reservoir 260b as intended.

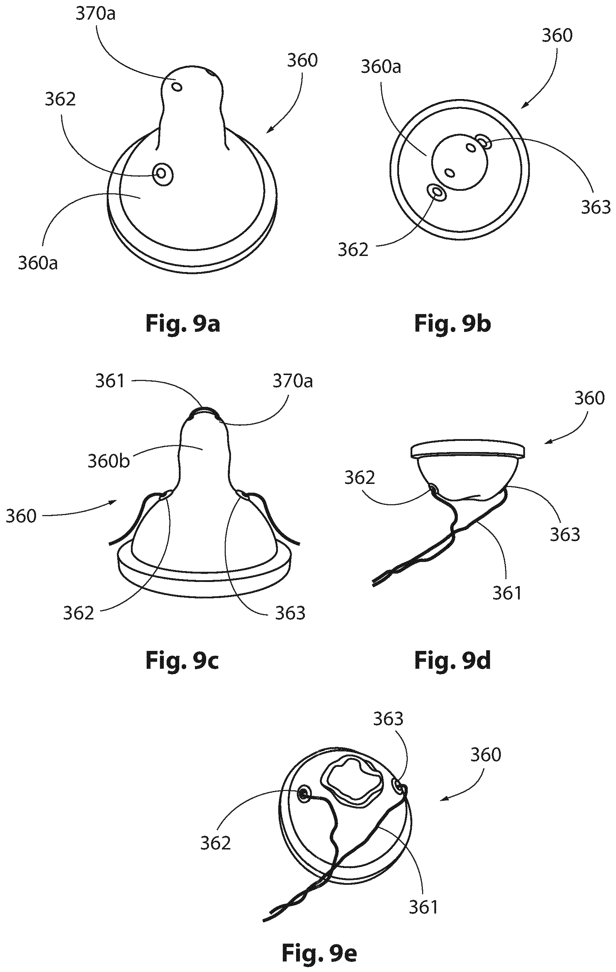

Another alternative implant 360 is now described with reference to Figs 9a-9e. The implant 360 is similar to the implant 260 with the exception that the string 361 is routed through the cap 360a rather than through the reservoir 360b as in the example implant 260 of Figs 8a-8d. To this end, the cap 360a comprises a first reinforced aperture 362 and a second reinforced aperture 363.

As shown in Fig 9c, the string 361 can be routed from the outside of the implant 360, through the first aperture 362, along the inside of the cap 360a and reservoir 360b to the proximal end 370a of the reservoir 360b. The string 361 then exits the reservoir 360b at the proximal 370a end and passes over an outside portion of the proximal end 370a before returning inside the reservoir 360b and running along the inside of the reservoir 360b and cap 360a before exiting again through the second aperture 363. The string 361 can be pulled in the same manner to as previously described with reference to Figs 8a and 8b, and similarly the string 361 may be attached to the rotatable knob or may be pulled separately by the user. Locating the first and second apertures 362, 363 on the cap 360a allows the reservoir 360b to be inverted as the proximal end 370a of the reservoir 360b is brought substantially in line with the first and second apertures 362, 363 in the cap 360a as shown in Figs 9d and 9e. Full inversion of the reservoir 360b ensures all semen in the reservoir 360b is delivered to the cap 360a, thereby increasing the chance of conception.

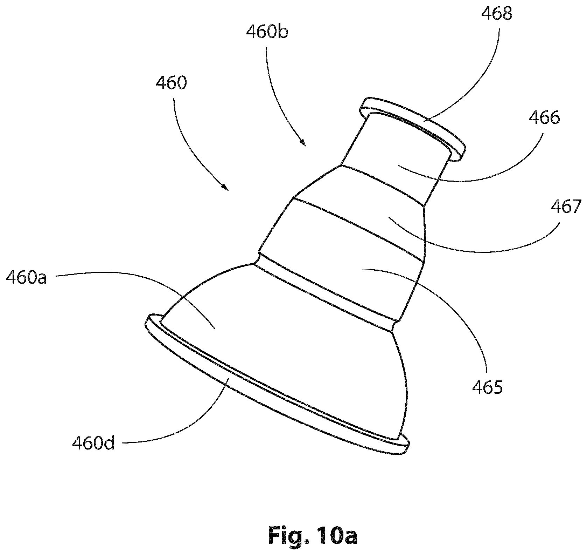

Another alternative implant 460 is now described with reference to Figs 10a-10d. The implant 460 is similar to the implants 160, 260, 360 in that the implant 460 comprises a cap 460a and a reservoir 460b. The cap 460a is sized and arranged to be positioned at the cervix, and is fluidly connected to the reservoir 460b which is arranged to hold semen and is sufficiently resilient such that semen can be expelled therefrom and into the cap 460a by deformation of the reservoir 460b when the implant 460 is installed adjacent the cervix, as will now be explained.

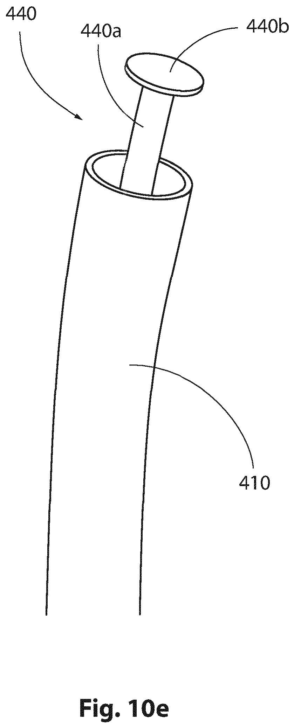

The implant 460 differs from the previously described implants 160, 260, 360 in that the reservoir 460b of the implant 460 comprises an upper reservoir section 465, a lower reservoir section 466, and an intermediate section 467. In a loading configuration, i.e. allowing semen to be loaded into the reservoir 460b, the upper reservoir section 465, lower reservoir section 466 and intermediate section 467 are fully expanded as shown in Fig 10a. Loaded semen is free to travel down through the upper reservoir section 465, intermediate reservoir section 467 and into the lower reservoir section 466. When the implant 460 is opened and the semen is expelled, the presently described arrangement allows the semen to be moved closer to the cervix and reliably ejects semen from the reservoir 460b without requiring pressing of the reservoir 460b by the vaginal walls. In this regard, in the presently described example, an implant actuator 440 (shown in Fig 10e) contacts the lower reservoir section 466 and causes compression of the lower reservoir section 466 and intermediate reservoir section 467. To assist in achieving the planned compression in use, the intermediate reservoir section 467 and/or the lower reservoir section 466 may be of a lighter and/or more compressible material than the upper reservoir section 465, thereby forcing the intermediate reservoir section 467 and lower reservoir section 466 to preferentially compress when acted on by the implant actuator 440. Said another way, it is preferable in the described example that the reservoir 460b is arranged such that a lower portion of the reservoir is compressed towards or into an upper portion of the reservoir to move the semen towards the cap 460a and ultimately towards the cervix in use. In some examples substantially all of the reservoir 460b may be compressible by the implant actuator 440. In some examples the reservoir 460b may be entirely compressed in use such that all semen in the reservoir 460b is ejected into the cap 460a. In other examples, only some of the reservoir 460b may be compressed by the implant actuator 440, thereby transferring the semen from one portion of the reservoir to another portion of the reservoir closer to the cervix.

In the presently described example the intermediate reservoir section 467 has a tapered profile in that the intermediate reservoir section 467 transitions from a larger diameter at its connection with the upper reservoir section 465 to a smaller diameter at its connection the lower reservoir section 466. Additionally, the lower reservoir section 466 comprises straight walls in the presently described example. The combined effect of the straight walls and tapered profile force the intermediate reservoir section 467 to preferentially collapse when a force is applied to the lower reservoir section 466, thereby moving the semen within the lower reservoir section 466 closer to the cervix before the collapsing of the lower reservoir section 466 itself. Upon collapsing of the lower reservoir section 466, semen may be ejected into the cap 460a or onto the cervix. Movement of the lower reservoir section 466 closer towards the cap 460a and cervix before compression of the lower reservoir section 466 allows the semen to be ejected straight out of the lower reservoir section 466 through the compressed intermediate reservoir section 467 and into either the cap 460a or onto the cervix, thereby improving the chances of the ejected semen reaching the cervix when compared with collapsing of the lower reservoir section 466 first, as the semen would need to be ejected with more force to reach the cervix and/or the cap 460a.

Still referring to Fig 10a, the lower reservoir section 466 further comprises a reservoir flange 468 which is a generally flat surface arranged to be abutted and pushed by a correspondingly flat actuator flange 440f (shown in Fig 10e) on the implant actuator 440. In use, the actuator flange 440f contacts the reservoir flange 468 and begins compression of the lower portion of the reservoir 460b to eject semen from the reservoir 460b.

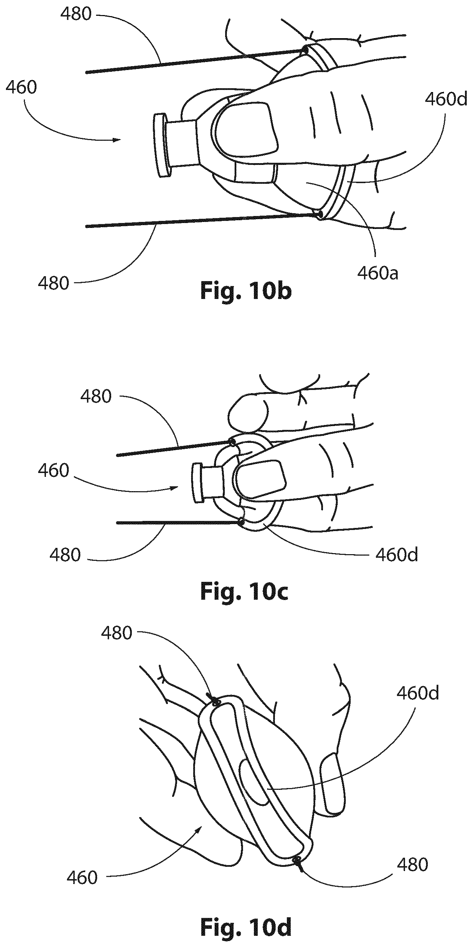

Although not shown in Fig 10a, the reservoir flange 468 may optionally comprise a retrieval thread to provide a means for the user to pull the implant 460 from outside of the vagina when the user wants to remove the implant 460. In this regard, the retrieval thread may be similar to the retrieval thread 180 shown in Fig 7 and comprise a similar bulb which can be gripped by the user when pulling the retrieval thread. In an alternative example, a retrieval thread may be attached to the cap 460a. The retrieval thread may be attached through the hemispherical walls of the cap 460a or attached to the rim 460d of the cap 460a. An example is shown in Fig 10b where a retrieval thread 480 is attached to the rim 460d of the cap 460a. The retrieval thread 480 may be a single thread or may be multiple threads. Attaching the retrieval thread 480 to the rim 460d of the cap 460a helps to break a seal created between the rim 460d and the cervix when the implant 460 is in use. As shown in Fig 10b, the retrieval thread 480 is connected at diametrically opposed points on the rim 460d to aid in breaking the seal created between the rim 460d and the cervix. This is achieved by the particular shape which the implant 460 adopts when the retrieval thread 480 is pulled in this manner when attached to the rim 460d at diametrically opposed points, as shown in Figs 10c and 10d.

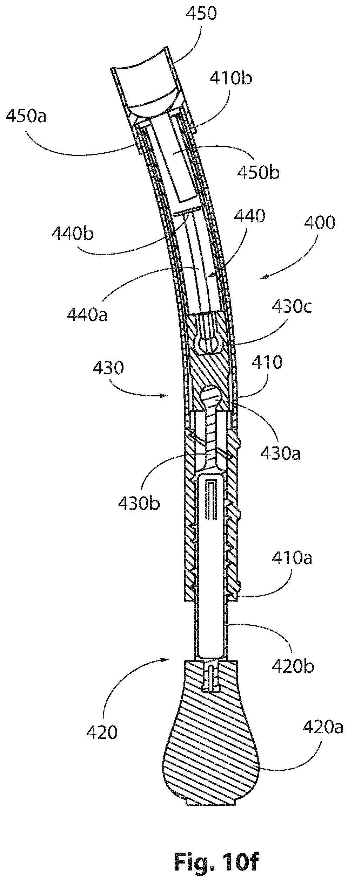

Referring now to Fig 10f, which shows the system 400 comprising the actuation mechanism described with reference to Fig 10e and for use with an implant 460 described with reference to Figs 10a-10d. The system 400 is shown in Fig. 10f in a loading configuration.

Similarly, to the system 100 described in Fig 1a, the system 400 comprises an elongate curved housing 410 in the form of an open-ended tubular. The housing 410 has a proximal end 410a and a distal end 410b. The housing 410 provides internal space within which the implant 460 (Figs 10a-10d) and other mechanisms can be safely housed and protected during insertion of the system 400 inside a vagina. The housing 410 has a slight curve to allow the distal end 410b to reach the cervix when the housing 410 is in the vagina.

The implant 460 (shown in Figs 10a-10d, not shown in Fig 10f) is securely enclosed within the housing 410 near to the distal end 410b of the housing 410.

The housing 410 has a release mechanism 420 attached at its proximal end 410a, which in the present embodiment is a rotatable knob 420a and a threaded member 420b. The rotatable knob 420a can optionally have a flat bottom or feet arranged such that the system 400 can stand upright freely. The system 400 may also comprise the previously mentioned angle of tilt which may be implemented in any of the previous described manners. It will be appreciated that the release mechanism 420 may be any other suitable device which can be easily operated be a user, such as a plunger or push button. Alternatively, as will be apparent to the reader, a rotatable hoop similar to the rotatable hoop 220a shown in Figs 1c-1e may be provided as the releasable mechanism 420. As previously described, the rotatable hoop may comprise a solid or hollow ring portion forming a central aperture. The ring portion may comprise a flat bottom which is configured to allow the system 400 to easily stand on a flat surface such as a bedside table, desk or work surface. A rotatable hoop provides a particularly convenient ergonomic grip which allows the user to place a finger through the central aperture or grip the outside of the ring portion. The hoop is again a particularly convenient shape to allow rapid injection moulding.

The threaded member 420b comprises thread projections or recesses which register with corresponding recesses or thread projections on the internal surface of the housing 410 toward its proximal end 410a. This arrangement allows the knob 420a to be rotated by a user to cause the threaded member 420b to travel into the housing 410 when the user wants to move the system 400 to an open configuration, i.e. when the distal end 410b of the housing 410 is located adjacent the cervix. The movement of the threaded member 420b as above described causes the implant 460 (shown in Figs 10a-10d, not shown in Fig 10f) to be moved out of the housing 410 by utilising a connection with an intermediate arrangement which is arranged to allow movement through the curved section of the housing 410.

In this regard, the system 400 comprises a ball-and-socket joint 430 to serve as the intermediate arrangement, wherein a ball 430a is connected by a shaft 430b to the threaded member 420b, to allow the travel of the threaded member 420b into the housing 410 to push the shaft 430b and the ball 430a. A corresponding socket 430c mates with the ball 430a to allow multi-directional movement. The socket 430c is attached to the implant actuator 440 described in Fig 10e which is shaped and arranged to slide within the housing 410 such that the implant actuator 440 will push the implant 460 out of the housing 410 as the user turns the rotatable knob 420a. As shown in more detail in Fig 10e, the implant actuator 440 is arranged to protrude from the distal end 410b of the housing 410 when the rotatable knob 420a is turned, thereby ensuring that the implant is pushed out of the housing 410.

Still referring to the system 400 in the loading configuration in Fig. 10f, the system 400 further comprises a removeable funnel 450 attached to the distal end of the housing 410. The funnel 450 is provided to allow efficient transfer of semen from outside of the system 400 to inside the system 400, and specifically, as will be described, directly to the implant 460.

The funnel 450 comprises a sleeve portion 450a and a channel portion 450b. The sleeve portion 450a is sized and arranged to register with the distal end 410b of the housing 410 such that the funnel 450 can be attached to the housing 410 via the sleeve portion 450a. The channel portion 450b is concentrically arranged with the sleeve portion 450a such that, as shown in Fig. 10f, when the sleeve portion 450a is attached to the distal end 410b of the housing 410, the channel portion 450b protrudes into the housing 410 and is concentric with the distal end 410b of the housing 410. The channel portion 450b may be substantially circular in cross-section or may be oval shaped. The sleeve portion 450a and the distal end 410b of the housing 410 may be configured to engage releasably in any known manner, such as, but not limited to, correspondingly registered threads or an interference fit.

In the loading configuration shown in Fig. 10f, the implant 460 is attached to the end of the channel portion 450b, such that the user can transfer semen to the implant 460 by pouring semen from a receptacle or ejaculating semen into the funnel 450.

The direct transfer of semen to the implant 460 held within the housing 410 mitigates the risk of losing all or part of a semen sample when compared with arrangements which require the implant 460 to be filled outside of the housing 410 and then the implant 460 transferred to the housing 410 for delivery to the cervix. The present invention overcomes accidental spillage of semen and/or total loss of a semen sample as the implant 460 is transferred to the housing 410. The present invention also greatly simplifies the loading operation by only requiring the user to provide a semen sample to the funnel 450 and then remove the funnel 450 from the housing 410.

Still referring to Fig 10f further details of the implant actuator 440 are now described. The implant actuator 440 comprises a connecting rod 440a and a flat actuator flange 440b. In use, when the rotatable knob 420a is turned, the connecting rod 440a is pushed upwards within the housing 410 until the flat actuator flange 440b contacts the reservoir flange 468 As the rotatable knob 420a is turned further, the flat actuator flange 440b compresses the reservoir 460b in the manner described above, thereby ejecting semen from the reservoir 460b into the cap 460a or onto the cervix.

Continued advancement of the connecting rod 440a is achieved by continued rotation of the rotatable knob 420a. The flat actuator flange 440b is advanced close to the distal end 410b of the housing 410, and pushes the implant 460 out of the housing 410 adjacent the cervix. In some examples, the system 400 may be configured such that the flat actuator flange 440b reaches the end of its journey of travel at or near the distal end 410b of the housing 410. In other examples, the system 400 may be configured such that the flat actuator flange 440b reaches the end of its journey of travel beyond the distal end 410b of the housing 400. In this regard, the flat actuator flange 440b may protrude from the housing 410, thereby ensuring the implant 460 is pushed completely out of the housing 410 and becomes separated from the housing 410. As shown in Fig 10f, the flat actuator flange 440b has a diameter which substantially fills the internal diameter of the housing 410, thereby ensuring that after compression of the reservoir 460b, a force is applied evenly across the implant 460 to push the implant 460 out from within the housing 410.

## Claims

1. A system (400) for assisted conception in a loading configuration, the system (400) comprising:
an implant (460);
an elongate curved housing (410);
a release mechanism (420) attached at a proximal end (410a) of the housing (410) and arranged to be operable by a user to deploy the implant (460) from the housing (410);
a translation mechanism (440) within the housing (410) arranged to translate a deployment operation of the release mechanism (420) to movement of the implant (460) out of the housing (410); and
a removable funnel (450) attached at a distal end (410b) of the housing (410) and arranged to deliver semen to the implant (460);
**characterized in that** the elongate curved housing (410) fully encloses the implant (40) and provides internal space within which the implant (460) can be safely housed and protected during insertion inside a vagina.

2. The system (400) of claim 1, wherein the system (400) is configured to move from the loading configuration in which semen can be loaded into the implant (460), through a delivery configuration in which the implant (460) is sealed closed, to an open configuration in which the implant (460) is separated from the housing (410) and is unsealed such that it can deliver semen to the cervix.

3. The system (400) of claim 1 or 2, wherein the implant (460) comprises a reservoir (460b) and a cap (460a), wherein the reservoir (460b) is attached to and in fluid communication with the cap (460a).

4. The system (400) of claim 3, wherein the reservoir (460b) is configured to be at least partially compressed by the translation mechanism (440), and the translation mechanism (440) is further arranged to translate the deployment operation of the release mechanism (420) to compression of at least a portion of the reservoir (460b).

5. The system (400) of claim 3 or 4, wherein the reservoir (460b) comprises a lower reservoir section (466), an intermediate reservoir section (467) and an upper reservoir section (465), wherein:
a. the intermediate reservoir section (467) is configured to be compressed by the translation mechanism (440) to move semen out of the reservoir (460b) or closer to the cervix; or
b. the intermediate reservoir section (467) and the lower reservoir section (466) are configured to be compressed by the translation mechanism (440) to move semen out of the reservoir (460b) or closer to the cervix; or
c. the lower reservoir section (466) is configured to be compressed by the translation mechanism (440) to move semen out of the reservoir (460b) or closer to the cervix.

6. The system (400) of claim 4 or 5, wherein the reservoir (460b) comprises a proximal end and a distal end, wherein the proximal end comprises a substantially flat flange portion (468) configured to be stiffer than a portion of the reservoir (460b), such that pressing of the flat flange portion (468) by the translation mechanism (440) causes compression of the portion of the reservoir (460b).

7. The system (400) of any preceding claim wherein the release mechanism (420) comprises:
a rotatable knob (420a) or rotatable hoop (220a).; and
a threaded member (420b);
wherein the threaded member (420b) is configured to register with a corresponding internal thread on the housing (410).

8. The system (400) of claim 7, wherein the translation mechanism (440) comprises:
a ball-and-socket joint (430); and
an implant actuator (440);
wherein the ball-and-socket joint (430) is connected on one side to the threaded member (420b) and on the other side to the implant actuator (440), and wherein the implant actuator (440) is arranged to move within the housing (410) into abutment with the implant (460) as the user turns the rotatable knob (420a) or rotatable hoop (220a).

9. The system (400) of claim 8 when claim 7 is dependent on any of claims 3 to 6, wherein the implant actuator (440) is further arranged to move within the housing (410) to compress at least a portion of the reservoir (460b) within the housing (410), thereby in use moving semen closer to the cervix or into the cap (460a).

10. The system (400) of claim 8 or 9, wherein the implant actuator (440) is further arranged to move within the housing (410) to push the implant (460) out from enclosure within the housing (410) as the user turns the rotatable knob (420a) or rotatable hoop (220a).

11. The system (400) of claim 10 when claim 7 is dependent on claim 3, wherein the cap (460a) is arranged to open and unseal when the implant (460) is pushed out from enclosure within the housing (410).

12. The system (400) of any of claims 1 to 11, configured such that the system (400) can stand upright on the release mechanism (420) and position the funnel (450) in a substantially vertical arrangement.

13. The system (400) of any of claims 7-11, wherein the threaded member (420b) and housing (410) are arranged such that their longitudinal axes are at an angle to each other, thereby causing an angle of tilt in the system (400) such that when the system (400) stands upright on the release mechanism (420), the funnel (450) is substantially vertical.

14. A method of arranging a system (400) for assisted conception, the method comprising the steps of:
providing an elongate curved housing (410) comprising a proximal end (410a) and a distal end (410b), wherein the elongate curved housing (410) provides internal space within which an implant can be safely housed and protected during insertion inside a vagina;
attaching a release mechanism (420) at the proximal end (410a) of the housing (410);
configuring the release mechanism (420) such that it can perform a deployment operation;
providing a resilient implant (460) comprising a cap (460a) and a reservoir (460b), wherein the cap (460a) comprises:
a rim (460d);
a shell;
a proximal end; and
a distal end
providing a removable funnel (450) comprising a sleeve (450a) and a channel portion (450b), wherein the sleeve (450a) and channel portion (450b) are configured concentrically to provide an annulus therebetween;
attaching the channel portion (450b) of the funnel (450) to the reservoir (460b) such that the channel portion (450b) and the reservoir (460b) are in fluid communication;
folding the resilient implant (460) in a clam shell arrangement such that two diametrically opposite points around the rim are folded toward the distal end of the cap (460a);
inserting the distal end of the cap (460a) into the annulus;
attaching the sleeve (450a) of the funnel (450) to the distal end of the housing (410) such that the implant (460) becomes fully enclosed within the housing (410); and
providing a translation mechanism (440) configured to translate a deployment operation of the release mechanism (420) to movement of the implant (460) out of the housing (410).

15. The method of claim 14, wherein the translation mechanism (440) is further configured to translate a deployment operation of the release mechanism (420) to compression of the reservoir (460b)

## Patentansprüche

1. System (400) zur Empfängnisunterstützung in einer Ladekonfiguration, wobei das System (400) umfasst:
ein Implantat (460);
ein längliches, gekrümmtes Gehäuse (410);
einen Auslösemechanismus (420), der an einem proximalen Ende (410a) des Gehäuses (410) angebracht und so angeordnet ist, dass er von einem Benutzer betätigt werden kann, um das Implantat (460) aus dem Gehäuse (410) zu entnehmen;
einen Umwandlungsmechanismus (440) innerhalb des Gehäuses (410), der so angeordnet ist, dass er einen Entnahmevorgang des Auslösemechanismus (420) in eine Bewegung des Implantats (460) aus dem Gehäuse (410) umwandelt; und
einen abnehmbaren Trichter (450), der an einem distalen Ende (410b) des Gehäuses (410) angebracht und so angeordnet ist, dass er Sperma zu dem Implantat (460) befördert;
**dadurch gekennzeichnet, dass** das längliche, gekrümmte Gehäuse (410) das Implantat (40) vollständig umschließt und einen Innenraum bereitstellt, in dem das Implantat (460) während des Einführens in eine Vagina sicher untergebracht und geschützt werden kann.

2. System (400) nach Anspruch 1, wobei das System (400) so konfiguriert ist, dass es sich von der Ladekonfiguration, in der Sperma in das Implantat (460) geladen werden kann, über eine Abgabekonfiguration, in der das Implantat (460) verschlossen ist, in eine offene Konfiguration bewegen kann, in der das Implantat (460) von dem Gehäuse (410) getrennt und nicht verschlossen ist, so dass es Sperma an den Gebärmutterhals abgeben kann.

3. System (400) nach Anspruch 1 oder 2, wobei das Implantat (460) einen Vorratsbehälter (460b) und eine Kappe (460a) umfasst, wobei der Vorratsbehälter (460b) an der Kappe (460a) angebracht ist und mit dieser in Fluidverbindung steht.

4. System (400) nach Anspruch 3, wobei der Vorratsbehälter (460b) so konfiguriert ist, dass er durch den Umwandlungsmechanismus (440) zumindest teilweise komprimiert wird, und der Umwandlungsmechanismus (440) weiter so angeordnet ist, dass er den Entnahmevorgang des Auslösemechanismus (420) in eine Kompression zumindest eines Teils des Vorratsbehälters (460b) umwandelt.

5. System (400) nach Anspruch 3 oder 4, wobei der Vorratsbehälter (460b) einen unteren Vorratsbehälterabschnitt (466), einen mittleren Vorratsbehälterabschnitt (467) und einen oberen Vorratsbehälterabschnitt (465) umfasst, wobei:
a. der mittlere Vorratsbehälterabschnitt (467) so konfiguriert ist, dass er durch den Umwandlungsmechanismus (440) komprimiert wird, um Sperma aus dem Vorratsbehälter (460b) heraus oder näher an den Gebärmutterhals heranzubewegen; oder
b. der mittlere Vorratsbehälterabschnitt (467) und der untere Vorratsbehälterabschnitt (466) so konfiguriert sind, dass sie durch den Umwandlungsmechanismus (440) komprimiert werden, um Sperma aus dem Vorratsbehälter (460b) heraus oder näher an den Gebärmutterhals heranzubewegen; oder
c. der untere Vorratsbehälterabschnitt (466) so konfiguriert ist, dass er durch den Umwandlungsmechanismus (440) komprimiert wird, um Sperma aus dem Reservoir (460b) heraus oder näher an den Gebärmutterhals heranzubewegen.

6. System (400) nach Anspruch 4 oder 5, wobei der Vorratsbehälter (460b) ein proximales Ende und ein distales Ende umfasst, wobei das proximale Ende einen im Wesentlichen flachen Flanschabschnitt (468) umfasst, der so konfiguriert ist, dass er steifer ist als ein Abschnitt des Vorratsbehälters (460b), so dass das Drücken des flachen Flanschabschnitts (468) durch den Umwandlungsmechanismus (440) eine Komprimierung des Abschnitts des Vorratsbehälters (460b) bewirkt.

7. System (400) nach einem der vorstehenden Ansprüche, wobei der Auslösemechanismus (420) umfasst:
einen drehbaren Knopf (420a) oder einen drehbaren Ring (220a);
und ein Gewindeelement (420b);
wobei das Gewindeelement (420b) so konfiguriert ist, dass es mit einem entsprechenden Innengewinde an dem Gehäuse (410) in Eingriff kommt.

8. System (400) nach Anspruch 7, wobei der Umwandlungsmechanismus (440) umfasst:
ein Kugelgelenk (430); und
einen Implantataktuator (440);
wobei das Kugelgelenk (430) auf einer Seite mit dem Gewindeelement (420b) und auf der anderen Seite mit dem Implantataktuator (440) verbunden ist, und wobei der Implantataktuator (440) so angeordnet ist, dass er sich innerhalb des Gehäuses (410) in Anlage an das Implantat (460) bewegt, wenn der Benutzer den drehbaren Knopf (420a) oder den drehbaren Ring (220a) dreht.

9. System (400) nach Anspruch 8, wenn Anspruch 7 von einem der Ansprüche 3 bis 6 abhängig ist, wobei der Implantataktuator (440) weiter so angeordnet ist, dass er sich innerhalb des Gehäuses (410) bewegt, um zumindest einen Abschnitt des Vorratsbehälters (460b) innerhalb des Gehäuses (410) zu komprimieren, wodurch im Gebrauch Sperma näher an den Gebärmutterhals oder in die Kappe (460a) bewegt wird.

10. System (400) nach Anspruch 8 oder 9, wobei der Implantataktuator (440) weiter so angeordnet ist, dass er sich innerhalb des Gehäuses (410) bewegt, um das Implantat (460) aus der Umhüllung innerhalb des Gehäuses (410) herauszudrücken, wenn der Benutzer den drehbaren Knopf (420a) oder den drehbaren Ring (220a) dreht.

11. System (400) nach Anspruch 10, wenn Anspruch 7 von Anspruch 3 abhängig ist, wobei die Kappe (460a) so angeordnet ist, dass sie sich öffnet und entsiegelt, wenn das Implantat (460) aus der Umhüllung innerhalb des Gehäuses (410) herausgedrückt wird.

12. System (400) nach einem der Ansprüche 1 bis 11, das so konfiguriert ist, dass das System (400) aufrecht auf dem Auslösemechanismus (420) stehen kann und den Trichter (450) in einer im Wesentlichen vertikalen Anordnung positionieren kann.

13. System (400) nach einem der Ansprüche 7-11, wobei das Gewindeelement (420b) und das Gehäuse (410) so angeordnet sind, dass ihre Längsachsen in einem Winkel zueinander stehen, wodurch ein Neigungswinkel in dem System (400) bewirkt wird, so dass, wenn das System (400) aufrecht auf dem Auslösemechanismus (420) steht, der Trichter (450) im Wesentlichen vertikal ist.

14. Verfahren zur Anordnung eines Systems (400) zur Empfängnisunterstützung, wobei das Verfahren die Schritte umfasst von:
Bereitstellen eines länglichen, gekrümmten Gehäuses (410), das ein proximales Ende (410a) und ein distales Ende (410b) umfasst, wobei das längliche, gekrümmte Gehäuse (410) einen Innenraum bereitstellt, in dem ein Implantat während des Einführens in eine Vagina sicher untergebracht und geschützt werden kann;
Anbringen eines Auslösemechanismus (420) an dem proximalen Ende (410a) des Gehäuses (410); Konfigurieren des Auslösemechanismus (420), so dass er einen Entnahmevorgang durchführen kann;
Bereitstellen eines elastischen Implantats (460), das eine Kappe (460a) und einen Vorratsbehälter (460b) umfasst, wobei die Kappe (460a) umfasst:
einen Rand (460d);
eine Schale;
ein proximales Ende; und
ein distales Ende
Bereitstellen eines abnehmbaren Trichters (450), der eine Hülle (450a) und einen Kanalabschnitt (450b) umfasst, wobei die Hülle (450a) und der Kanalabschnitt (450b) konzentrisch konfiguriert sind, um dazwischen einen Ringraum bereitzustellen;
Anbringen des Kanalabschnitts (450b) des Trichters (450) an dem Vorratsbehälter (460b), so dass der Kanalabschnitt (450b) und der Vorratsbehälter (460b) in Fluidverbindung stehen;
Falten des elastischen Implantats (460) in einer Muschelschalenanordnung, so dass zwei diametral gegenüberliegende Punkte um den Rand herum in Richtung des distalen Endes der Kappe (460a) gefaltet werden;
Einführen des distalen Endes der Kappe (460a) in den Ringraum;
Anbringen der Hülle (450a) des Trichters (450) an dem distalen Ende des Gehäuses (410), so dass das Implantat (460) vollständig innerhalb des Gehäuses (410) eingeschlossen wird; und
Bereitstellen eines Umwandlungsmechanismus (440), der so konfiguriert ist, dass er einen Entnahmevorgang des Auslösemechanismus (420) in eine Bewegung des Implantats (460) aus dem Gehäuse (410) heraus umwandelt.

15. Verfahren nach Anspruch 14, wobei der Umwandlungsmechanismus (440) weiter so konfiguriert ist, dass er einen Entnahmevorgang des Auslösemechanismus (420) in eine Komprimierung des Vorratsbehälters (460b) umwandelt.

## Revendications

1. Système (400) pour la conception assistée dans une configuration de chargement, le système (400) comprenant :
un implant (460) ;
un boîtier (410) incurvé allongé ;
un mécanisme de libération (420) fixé à une extrémité proximale (410a) du boîtier (410) et agencé pour pouvoir être commandé par un utilisateur pour déployer l'implant (460) depuis le boîtier (410) ;
un mécanisme de translation (440) à l'intérieur du boîtier (410) agencé pour translater une opération de déploiement du mécanisme de libération (420) en déplacement de l'implant (460) hors **du** boîtier (410) ; et
un entonnoir (450) amovible fixé à une extrémité distale (410b) du boîtier (410) et agencé pour délivrer du sperme à l'implant (460) ;
**caractérisé en ce que** le boîtier (410) incurvé allongé enveloppe entièrement l'implant (40) et fournit un espace interne à l'intérieur duquel l'implant (460) peut être logé et protégé en toute sécurité pendant l'insertion à l'intérieur d'un vagin.

2. Système (400) selon la revendication 1, dans lequel le système (400) est configuré pour se déplacer de la configuration de chargement, dans laquelle du sperme peut être chargé dans l'implant (460), à une configuration de délivrance dans laquelle l'implant (460) est fermé de manière scellée, puis à une configuration ouverte dans laquelle l'implant (460) est séparé du boîtier (410) et n'est pas scellé, de telle sorte qu'il puisse délivrer du sperme au col de l'utérus.

3. Système (400) selon la revendication 1 ou la revendication 2, dans lequel l'implant (460) comprend un réservoir (460b) et un capuchon (460a), dans lequel le réservoir (460b) est fixé au capuchon (460a) en communication fluidique avec celui-ci.

4. Système (400) selon la revendication 3, dans lequel le réservoir (460b) est configuré pour être au moins partiellement comprimé par le mécanisme de translation (440), et le mécanisme de translation (440) est en outre agencé pour translater l'opération de déploiement du mécanisme de libération (420) en compression d'au moins une partie du réservoir (460b).

5. Système (400) selon la revendication 3 ou la revendication 4, dans lequel le réservoir (460b) comprend une section de réservoir inférieure (466), une section de réservoir intermédiaire (467) et une section de réservoir supérieure (465), dans lequel :
a. la section de réservoir intermédiaire (467) est configurée pour être comprimée par le mécanisme de translation (440) pour déplacer le sperme hors du réservoir (460b) ou le rapprocher du col de l'utérus ; ou
b. la section de réservoir intermédiaire (467) et la section de réservoir inférieure (466) sont configurées pour être comprimées par le mécanisme de translation (440) pour déplacer le sperme hors du réservoir (460b) ou le rapprocher du col de l'utérus ; ou
c. la section de réservoir inférieure (466) est configurée pour être comprimée par le mécanisme de translation (440) pour déplacer le sperme hors du réservoir (460b) ou le rapprocher du col de l'utérus.

6. Système (400) selon la revendication 4 ou la revendication 5, dans lequel le réservoir (460b) comprend une extrémité proximale et une extrémité distale, dans lequel l'extrémité proximale comprend une partie bride (468) sensiblement plate configurée pour être plus rigide qu'une partie du réservoir (460b), de sorte que le pressage de la partie bride (468) plate par le mécanisme de translation (440) entraîne la compression de la partie du réservoir (460b).

7. Système (400) selon une quelconque revendication précédente dans lequel le mécanisme de libération (420) comprend :
un bouton rotatif (420a) ou un anneau rotatif (220a) ; et
un organe fileté (420b) ;
dans lequel l'organe fileté (420b) est configuré pour s'aligner avec un filetage interne correspondant sur le boîtier (410).

8. Système (400) selon la revendication 7, dans lequel le mécanisme de translation (440) comprend :
une articulation à rotule (430) ; et
un actionneur (440) d'implant ;
dans lequel l'articulation à rotule (430) est reliée d'un côté à l'organe fileté (420b) et de l'autre côté à l'actionneur (440) d'implant, et dans lequel l'actionneur (440) d'implant est agencé pour se déplacer à l'intérieur du boîtier (410) pour venir en butée avec l'implant (460) lorsque l'utilisateur tourne le bouton rotatif (420a) ou l'anneau rotatif (220a).

9. Système (400) selon la revendication 8 lorsque la revendication 7 dépend de l'une quelconque des revendications 3 à 6, dans lequel l'actionneur (440) d'implant est en outre agencé pour se déplacer à l'intérieur du boîtier (410) pour comprimer au moins une partie du réservoir (460b) à l'intérieur du boîtier (410), rapprochant ainsi le sperme du col de l'utérus ou l'amenant dans le capuchon (460a) lors de son utilisation.

10. Système (400) selon la revendication 8 ou la revendication 9, dans lequel l'actionneur (440) d'implant est en outre agencé pour se déplacer à l'intérieur du boîtier (410) pour pousser l'implant (460) hors de l'enceinte à l'intérieur du boîtier (410) lorsque l'utilisateur tourne le bouton rotatif (420a) ou l'anneau rotatif (220a).

11. Système (400) selon la revendication 10 lorsque la revendication 7 dépend de la revendication 3, dans lequel le capuchon (460a) est agencé pour s'ouvrir et ne plus être scellé lorsque l'implant (460) est poussé hors de l'enceinte à l'intérieur du boîtier (410).

12. Système (400) selon l'une quelconque des revendications 1 à 11, configuré de telle sorte que le système (400) puisse se tenir debout sur le mécanisme de libération (420) et positionner l'entonnoir (450) dans un agencement sensiblement vertical.

13. Système (400) selon l'une quelconque des revendications 7 à 11, dans lequel l'organe fileté (420b) et le boîtier (410) sont agencés de sorte que leurs axes longitudinaux sont à angle l'un par rapport à l'autre, provoquant ainsi un angle d'inclinaison dans le système (400) de sorte que, lorsque le système (400) se tient debout sur le mécanisme de libération (420), l'entonnoir (450) est sensiblement vertical.

14. Procédé d'agencement d'un système (400) pour la conception assistée, le procédé comprenant les étapes de :
fourniture d'un boîtier (410) incurvé allongé comprenant une extrémité proximale (410a) et une extrémité distale (410b), dans lequel le boîtier (410) incurvé allongé fournit un espace interne à l'intérieur duquel un implant peut être logé et protégé en toute sécurité pendant l'insertion à l'intérieur d'un vagin ;
fixation d'un mécanisme de libération (420) à l'extrémité proximale (410a) du boîtier (410) ; configuration du mécanisme de libération (420) de sorte qu'il puisse effectuer une opération de déploiement ;
fourniture d'un implant élastique (460) comprenant un capuchon (460a) et un réservoir (460b), dans lequel le capuchon (460a) comprend :
un rebord (460d) ;
une coque ;
une extrémité proximale ; et
une extrémité distale
fourniture d'un entonnoir (450) amovible comprenant un manchon (450a) et une partie canal (450b), dans lequel le manchon (450a) et la partie canal (450b) sont configurés de manière concentrique pour fournir un espace annulaire entre eux ;
fixation de la partie canal (450b) de l'entonnoir (450) au réservoir (460b) de sorte que la partie canal (450b) et le réservoir (460b) soient en communication fluidique ;
pliage de l'implant (460) élastique dans un agencement de coque à pince de sorte que deux points diamétralement opposés autour du rebord soient pliés vers l'extrémité distale du capuchon (460a) ;
insertion de l'extrémité distale du capuchon (460a) dans l'espace annulaire ;
fixation du manchon (450a) de l'entonnoir (450) à l'extrémité distale du boîtier (410) de sorte que l'implant (460) soit complètement enfermé à l'intérieur du boîtier (410) ; et
fourniture d'un mécanisme de translation (440) configuré pour translater une opération de déploiement du mécanisme de libération (420) en mouvement de l'implant (460) hors du boîtier (410).

15. Procédé selon la revendication 14, dans lequel le mécanisme de translation (440) est en outre configuré pour translater une opération de déploiement du mécanisme de libération (420) en compression du réservoir (460b)
